# EUROPÄISCHE PATENTSCHRIFT

(11) **EP 1 400 829 B1**
(45) Veröffentlichungstag und Bekanntmachung des Hinweises auf die Patenterteilung: **31.01.2007**
(21) Anmeldenummer: 03019901.2
(22) Anmeldetag: 02.09.2003
(51) Int. Cl.: G02B 21/00, G02B 21/22, A61B 3/13

(54) **Ophtalmo-Operationsmikroskop mit Objektbeleuchtung**
Microscope for ophthalmologic surgery with object illumination
Microscope pour chirurgie ophthalmologique avec éclairage de l'objet

(30) Priorität: 17.09.2002 DE 10242983
(43) Veröffentlichungstag der Anmeldung: 24.03.2004
(73) Patentinhaber: Leica Microsystems (Schweiz) AG, 9435 Heerbrugg (CH)
(72) Erfinder: Pensel, Jürgen, Dr., 9450 Altstätten (CH); Sander, Ulrich, Dr., 9445 Rebstein (CH)
(74) Vertreter: Rosenich, Paul

(56) Entgegenhaltungen:
- US-A- 4 786 154
- US-A- 5 619 372
- US-A- 6 065 837
- US-B1- 6 350 031

## Beschreibung

Die Erfindung betrifft ein Ophthalmo-Operationsmikroskop mit einer Objektbeleuchtung mit speziellen optischen Eigenschaften.

Verschiedenste Arten von Beleuchtungen, die in ein Operationsmikroskop integriert sind, sind dem Fachmann hinlänglich bekannt. Diese bekannten Beleuchtungen werden entweder seitlich am Mikroskop als Schrägbeleuchtung, beispielsweise mit einem Schwanenhals, vgl. den WILD-Prospekt "Schrägleuchte", M667d-VI.86, Publikationsdatum: Juni 1986, fixiert oder aber zur Verringerung des Beleuchtungswinkels direkt in das Stereomikroskop eingebaut, so dass der Beleuchtungsstrahlengang durch das Hauptobjektiv geführt wird (EP-B1-0 661 020). Allen diesen Beleuchtungen ist gemeinsam, dass sie als Beleuchtungslicht Weitsicht verwenden. In der Regel werden Halogenlampen als Lichtquellen verwendet. Eine spektrale Selektion des Weißlichts erfolgt gewöhnlich zum Schutz des menschlichen Auges gegenüber schädlicher Strahlung. Diese schädliche Strahlung wird in der Regel mittels UV- und IR-Filtern absorbiert.

Bei den heutigen Katarakt-Operationen ist es notwendig, die hintere Linsenkapsel des Patientenauges und darauf verbliebene Linsenreste mit dem Mikroskop kontrastreich zu sehen. Dazu wird der hinlänglich bekannte "Red Reflex" verwendet. Die Erzeugung des Red Reflexes ist technisch nur sehr aufwändig über die sogenannte 0°- oder Koaxial-Technik realisierbar. Diese Beleuchtungen können zu störenden Vignettierungen und Reflexen im Beobachtungsstrahlengang führen.

Durch die Art der Entstehung des Red Reflexes ist er stark vom einzelnen Patientenauge abhängig und reagiert sehr sensibel auf Bewegungen des Auges. Weiterhin bedingt die Art der Entstehung des Red Reflexes, dass der Beobachter im Assistenten-Beobachterstrahlengang einen anderen Red Reflex sieht als der Beobachter im Hauptstrahlengang.

Diese Nachteile können durch eine geeignete Beleuchtungsanordnung im Mikroskop ausgeglichen werden, vgl. hierzu den Leica-Prospekt "The Leica Imaging Module", Dokumenten-Nr.: 10 M1 410 1de - X.99.RDV, Drucklegung: Oktober 1999.

Aus dem Stand der Technik ist weiter bekannt, dass bei Labormikroskopen Polarisations- und/oder Phasen- und/oder Farbfilter bei der visuellen Betrachtung eingesetzt werden, um Objektstrukturen besser sichtbar zu machen, vgl. hierzu: K. Michel: "Die Grundzüge der Theorie des Mikroskops", Stuttgart 1981. Die Technik des Phasenkontrasts wird auch im Ophthalmo-Operationsmikroskop verwendet, US-A-5 619 372 und US-B1-6 350 031.

Als weitere Verbesserung ist bekannt, dass zur Sichtbarmachung verschiedener Medien des menschlichen Auges präoperative Färbungen dieser Medien durchgeführt werden, vgl. hierzu den Farbstoff "Vision Blue" der Medizintechnik-Vertriebs-GmbH, Mömbris.

Färbungen einzelner Medien in vivo am Patientenauge sind insofern schwierig, als sie wegen fehlender Perfusion der optischen Augenmedien lange präoperativ und invasiv erfolgen müssen, kaum selektiv sind und sich zusätzlich im Laufe der Operation verändern können. Dieser Prozess führt zu zusätzlichen Belastungen des Patientenauges. Präoperative Färbungen können während der Operation nur ungenügend gesteuert werden, so dass es nicht möglich ist, für verschiedene Operationsphasen den Schwerpunkt der Sichtbarmachung im Mikroskop auf verschiedene Medien oder Grenzschichten der Medien im Auge zu legen.

Zudem ist die Einspiegelung von Informationen in den Strahlengang eines Operations-Mikroskops aus der US-A-4 786 154 bekannt.

Der Erfindung liegt nun die Aufgabe zugrunde, eine Lösung für diese Probleme zu finden und die Sichtbarkeit der verschiedenen Schichten und Medien im Auge zu verbessern.

Gelöst wird diese Aufgabe durch ein Ophthalmo-Operationsmikroskop gemäß Anspruch 1, welches die Erkennung und Unterscheidung der einzelnen Medien im Auge und/oder der einzelnen Grenzschichten zwischen den Medien ermöglicht. Dies ohne die obengenannten Nachteile der heute verwendeten Verfahren wie 'Red Reflex' oder 'präoperative Färbungen'.

Der Erfindung liegt die aus der Physik bekannte Tatsache zugrunde, dass Licht mit unterschiedlichen optischen Eigenschaften (Spektrum und/oder Polarisation und/oder Phase) unterschiedlich in einzelnen Medien und/oder an deren Grenzschichten reflektiert, absorbiert oder gestreut wird. Die Gründe hierfür sind die verschiedenen physikalisch-chemischen Zusammensetzungen beziehungsweise die unterschiedliche Morphologie dieser Medien. Die Nutzung dieses physikalischen Effekts erfolgt erfindungsgemäß auf die folgende Art und Weise:

Es wird mittels der erfindungsgemäßen Beleuchtung Licht mit speziellen optischen Eigenschaften erzeugt. Dies kann einerseits durch eine spektrale Selektion des Weißlichts, andererseits durch eine unterschiedliche Polarisation oder Phase des Lichts oder eine Kombination dieser Faktoren erfolgen. Diese optischen Eigenschaften des Beleuchtungslichts können auf verschiedene Art und Weise erreicht werden:

Zum besseren Verständnis werden nachfolgend bereits Bezugszeichen angegeben, die in der Lösungsbeschreibung aufgeführt sind.

Vor die Lichtquelle 7 werden Farb- und/oder Polarisationsfilter 18 eingesetzt, die das Licht in der gewünschten Weise verändern. Andererseits können auch Lichtquellen 7 verwendet werden, die aufgrund ihrer Lichterzeugung schon die gewünschten spektralen Eigenschaften aufweisen. Solche Lichtquellen sind zum Beispiel Gasentladungslampen.

Dieses mit speziellen optischen Eigenschaften ausgestattete Beleuchtungslicht, insbesondere auch im nicht sichtbaren Bereich, wird auf dem hinlänglich bekannten Weg mit einem Umlenkelement 21 für die Beleuchtung, beispielsweise einem Spiegel oder Prisma, in das Stereomikroskop 1 mit Vergrößerungssystem eingekoppelt und sodann über das Objektiv 3 des Stereomikroskops 1 auf das Patientenauge 5 geleitet. Die unterschiedlichen Medien im Patientenauge 5 bewirken aufgrund der oben genannten physikalisch-chemischen Eigenschaften eine unterschiedliche Reflexion, Absorption, Streuung oder andersartige Wechselwirkungen des Beleuchtungslichts an den einzelnen Medien und/oder an deren Grenzschichten.

Das derart veränderte Licht wird mit einem teildurchlässigen Umlenkelement 10, das ein Spiegel oder Prisma sein kann, aus dem Beobachtungsstrahlengang 2 des Stereomikroskops 1 ausgekoppelt. Hierzu werden bekannte optische Teiler verwendet. Anschließend wird das Licht einem Sensor 12 zugeführt, der in der Lage ist, das in verschiedenen Medien des Auges und/oder an deren Grenzschichten unterschiedlich veränderte Licht zu detektieren und in entsprechende elektrische und/oder elektronische Signale umzuwandeln.

Diese Signale werden auf eine Auswertungseinheit 14, beispielsweise einen Computer, geführt, der in der Lage ist, die eintreffenden Signale nach ihrer Veränderung auszuwerten und ein Ansteuersignal 15 für einen Projektor 16 beziehungsweise einen Monitor oder ein Display zu erzeugen. Der Projektor 16 generiert aufgrund dieses Ansteuersignals 15 ein elektronisch erzeugtes optisches Bild. Das mittels des Projektors 16 beziehungsweise des Monitors oder des Displays erzeugte optische Bild wird über ein bekanntes UmlenkElement 9 in den Beobachtungsstrahlengang 2 eingekoppelt und dem direkt durch das Stereomikroskop 1 sichtbare Abbild des Objekts wahlweise überlagert.

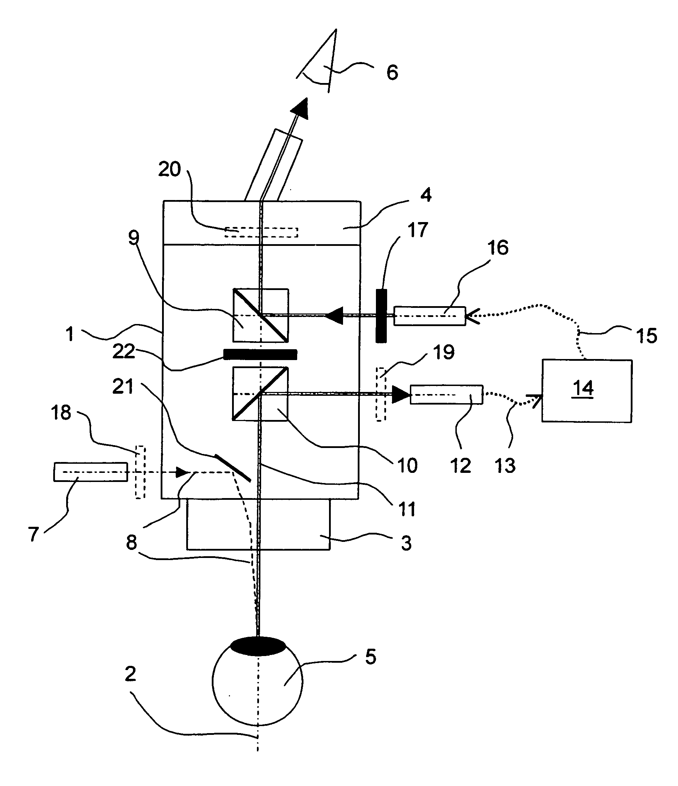

Der zweite Beobachtungsstrahlengang des Stereomikroskops 1 ist der Einfachheit halber in der Zeichnung nicht dargestellt, da er in der Projektion hinter dem Beobachtungsstrahlengang 2 liegt.

Für eine stereoskopische Dateneinspiegelung, wie sie beispielsweise aus der EP -B1- 1 008 005 bekannt ist, werden zwei separate Umlenkelemente 10 und zwei separate Sensoren 12 für das rechte und das linke Bild mit zwei entsprechenden Umlenkelementen 9 in den rechten und linken Beobachtungsstrahlengang des Stereomikroskops 1 benötigt. Verfahren zur Dateneinspiegelung sind bekannt, vgl. hierzu den bereits erwähnten den Leica-Prospekt: "The Leica Imaging Module".

Mit Hilfe von Dateneinspiegelungen wurden allerdings bisher immer Bilder eingespiegelt, die von Fremdgeräten wie Endoskopen, Computern oder Videokameras erzeugt wurden.

Bei der hier beschriebenen erfinderischen Idee wird jedoch ein Bild in das Stereomikroskop 1 eingespiegelt, das von demselben Stereomikroskop 1 aufgenommen, aber in seinem Bildinhalt verändert wurde.

Durch eine geeignete Wahl der Position des eingespiegelten Bildes (Lage und Gestalt des Zwischenbildes im nicht näher dargestellten Okular) kann es direkt und passgenau dem direkt durch das Stereomikroskop 1 optisch erzeugten Bild des Patientenauges 5 überlagert werden.

In den Beobachtungsstrahlengang 2 des Stereomikroskops 1 beziehungsweise den Strahlengang des eingespiegelten Bildes sind zusätzliche Shutter 22,17 eingebaut, die es ermöglichen, wahlweise das ursprüngliche Stereomikroskopbild, das veränderte eingespiegelte Bild oder ein Überlagerung beider Bilder zu sehen. Als Projektor 16, Monitor oder Display können beispielsweise Echtfarben-, Falschfarben- oder Schwarz/Weiß-Systeme eingesetzt werden.

Um die oben genannten optischen Effekte zu erreichen, sind beliebige Kombinationen zwischen spektraler Selektion, Polarisation und/oder Phase des Beleuchtungslichts beziehungsweise beliebige Kombinationen zwischen Echtfarben-, Falschfarben- und Schwarz/Weiß-Projektion auszuwählen. Im Extremfall kann auch mit reinem "Falschfarbenlicht" zum Schutz des Patienten- und Beobachterauges gearbeitet werden.

Damit werden die folgenden Verbesserungen bezüglich der herkömmlichen Systeme und Verfahren erreicht:

Die einzelnen Medien des menschlichen Auges und/oder deren Grenzschichten werden dem Chirurgen in Art, Form und Lage in korrekter, mit dem Original übereinstimmender Weise sichtbar gemacht. Zudem ist der Chirurg in der Lage, speziell von ihm gewünschte Elemente des Auges, beispielsweise die Linse, durch eine geeignete Wahl der optischen Eigenschaften des Beleuchtungslichts (spektrale Selektion, Polarisation und/oder Phase und/oder der nachfolgenden Bildbearbeitung) hervorzuheben oder zu unterdrücken. Die Sichtbarkeit der betrachteten Medien und/oder Grenzschichten ist mit dieser neuen Methode - im Gegensatz zur Beobachtung mit dem Red Reflex - unabhängig von der Bewegung des Patientenauges.

Als vereinfachte Variante kann eine vergleichbare Visualisierung von Art, Form und Lage einzelner Medien des Patientenauges und/oder deren Grenzschichten auch ohne Ein- und Ausspiegelung und elektronische Bearbeitung auf rein visueller Basis erreicht werden, wenn die Veränderung des Beleuchtungslichts im sichtbaren Bereich erfolgt. Dazu werden Filter in die Beleuchtung und an einem geeigneten Ort zwischen Vergrößerungssystem und Okularen des Stereomikroskops eingesetzt, die die gewünschte spektrale Selektion und damit die Sichtbarmachung der Struktur der beobachteten Medien erzeugen.

In der Zeichnung wird eine erfindungsgemäße Objektbeleuchtung für die Beobachtung in einem Ophthalmo-Operationsmikroskop schematisch dargestellt.

Darin ist ein Stereomikroskop 1 mit einem Vergrößerungssystem, einem der beiden Beobachtungsstrahlengänge 2, einem Objektiv 3, einem Tubus 4 mit Okularen sowie ein Patientenauge 5 und ein Beobachter 6 dargestellt. Die in das Stereomikroskop 1 integrierte Beleuchtung und die Abbildung des Patientenauges 5 sind erfindungsgemäß wie folgt aufgebaut:

Am Stereomikroskop 1 ist eine Lichtquelle 7 angebracht. Das von dieser Lichtquelle 7 erzeugte Licht wird über einen zuschaltbaren Filter 18 für die Beleuchtung auf ein Umlenkelement 21 geleitet und von dort auf das Patientenauge 5 projiziert. Das vom Patientenauge 5 veränderte und reflektierte Beleuchtungslicht wird mittels eines Umlenkelements 10 aus dem Beobachtungsstrahlengang 2 des Stereomikroskops 1 ausgekoppelt und über wahlweise zuschaltbare optische Filter 19 für einen lichtempfindlichen Sensor 12 auf diesen geleitet. Der Sensor 12 detektiert das am Patentenauge 5 reflektierte Licht nach Art, Form und Lage und wandelt es in elektrische beziehungsweise elektronische Signale 13 um.

Diese Signale 13 werden mit einer Auswertungseinheit 14, beispielsweise einem Computer, in Ansteuersignale 15 für einen Projektor 16 umgewandelt und an diesen weitergeleitet. Der Projektor 16 erzeugt aus den ihm zugeleiteten Ansteuersignalen 15 ein optisches Bild, welches mittels eines Umlenkelements 9 in den Beobachtungsstrahlengang 2 des Stereomikroskops 1 eingekoppelt wird. Das eingekoppelte Bild wird wahlweise mit Shuttern 17 und 22 dem direkt durch das Stereomikroskop 1 erzeugten optischen Bild des Patientenauges 5 überlagert.

Aus der Zeichnung ist weiter ersichtlich, dass der Shutter 22 zwischen den beiden Umlenkelementen 9 und 10 im Beobachtungsstrahlengang 2 des Stereomikroskops 1 angebracht ist. Der Shutter 17 ist im Strahlengang des vom Projektor 16 erzeugten optischen Bildes angebracht. Durch diese Shutter 17 und 22 lassen sich verschiedene Überlagerungs-Kombinationen des direkt vom Stereomikroskop 1 optisch erzeugten Bildes des Patientenauges 5 und des vom Projektor 16 erzeugten optischen Bildes herstellen.

Die Auswertungseinheit 14 ist in der Lage, mit Hilfe einer Kalibriervorrichtung das einzukoppelnde Bild hinsichtlich seiner Lage und Größe deckungsgleich dem direkt durch das Stereomikroskop 1 optisch erzeugten Bild des Patientenauges 5 zu überlagern. Damit wird eine einfache Erkennbarkeit der Lage, der Form und der Größe der unterschiedlichen Medien und Grenzschichten des Patientenauges 5 ermöglicht.

Als vereinfachte Variante kann eine vergleichbare Visualisierung von Art, Form und Lage einzelner Medien und/oder Grenzschichten des Patientenauges 5 ohne Ein- und Ausspiegelung und elektronische Bearbeitung auf rein visueller Basis erreicht werden, wenn die gewünschten Veränderungen des Beleuchtungslichts im sichtbaren Bereich erfolgen. Dazu werden Filter 18 in den Beleuchtungsstrahlengang 8 und an einem geeigneten Ort zwischen dem nicht dargestellten Vergrößerungssystem und den Okularen des Tubus 4, vergleiche den Filter 20, eingesetzt.

In dieser vereinfachten Variante erhält ein in der Zeichnung nicht dargestellter Assistentenbeobachter das gleiche Bild wie der Hauptbeobachter mit der verbesserten Darstellung der Medien und/oder deren Grenzschichten des Patientenauges 5, wenn entsprechend zusätzliche Filter im Assistententubus vor den Okularen eingesetzt werden.

### Bezugszeichenliste

- 1: Stereomikroskop mit Vergrößerungssystem
- 2: Beobachtungsstrahlengang von (1)
- 3: Objektiv
- 4: Tubus mit Okularen
- 5: Patientenauge
- 6: Beobachter
- 7: Lichtquelle(n)
- 8: Strahlengang des Beleuchtungslichts
- 9: Umlenkelement
- 10: Umlenkelement
- 11: reflektiertes Beleuchtungslicht
- 12: Sensor
- 13: elektrisches Signal von (12)
- 14: Auswertungseinheit (Computer)
- 15: Ansteuersignal für (16)
- 16: Projektor
- 17: Shutter
- 18: Farb- u./o. Polarisations-Filter (zuschaltbar) für die Beleuchtung
- 19: Filter (zuschaltbar) für (12)
- 20: Filter (zuschaltbar) für direkte Beobachtung
- 21: Umlenkelement für die Beleuchtung
- 22: Shutter

## Patentansprüche

1. Ophthalmo-opemtionsmikroskop (1) zur Beobachtung eines Patientenauges (5), wobei das Ophthalmo-Operationsmikroskop (1) ein Objektiv (3), ein Vergrößerungssystem, einen Tubus mit Okularen (4), mindestens einen Beobachtungs-Strahlengang (2) aufweist, der vom Patientenauge (5) durch das Objektiv (3) und durch den Tubus mit Okularen (4) einem Beobachter (6) zugeführt ist
und wobei an dem Ophthalmo-Operationsmikroskop (1) eine Lichtquelle (7) angeordnet ist, deren Beleuchtungs-Strahiengang (8) mittels eines Umlenkelements (21) und dem Objektiv (3) so umlenkbar ist, dass der Beleuchtungs-Strahlengang (8) mit dem Beobachtungs-Strahlengang (2) auf das Patientenauge (5) auftrifft,
**dadurch gekennzeichnet, dass**
mindestens ein erster Spektral-, Farb- oder Polarisationsfilter (18) oder Kombinationen hiervon im Beleuchtungs-Strahlengang (8) und mindestens ein zweiter Spektral-, Farb- oder Polarisationsfilter (20) zwischen dem Vergrößerungssystem und dem Tubus mit Okularen (4) oder im Tubus mit Okularen (4) selbst zuschaltbar angeordnet sind, sodass die unterschiedlichen Medien des Patientenauges (5) unterschiedlich darstellbar sind und dass ein üchtempfindlicher Sensor (12) für das elektronische Detektieren des vom Patientenauge (5) reflektierten und über ein Umlenkelement (10) aus dem Beobachtungs-Strahlengang (2) ausgekoppelten Beleuchtungslichts vorgesehen ist, mittels dem das reflektierte Beleuchtungslicht in entsprechende elektronische Signale (13) umgesetzt und an eine Auswertungseinheit (14) weitergeleitet werden, mittels derer die elektronischen Signale des Sensors (12) ausgewertet werden können;
und an einen Projektor (16), Monitor oder Display weiterleitbar ist, der im Betriebszustand durch ein Ansteuersignal (15) der Auswertungseinheit (14) so angesteuert werden kann, dass ein Bild erzeugt wird, das über ein Umlenkelement (9) wiederum in den Beobachtungs-Strahlengang (2) des Ophthalmo-Operationsmikroskops (1) einspiegelbar ist und dass im Beobachtungs-Strahlengang (2) des Ophthalmo-Operationsmikroskops (1) und im Strahlengang des eingekoppelten Bildes Shutter (17, 22) angeordnet sind, die im Betriebszustand wahlweise das direkte Beobachtungslicht oder das eingespiegelte Licht abschalten.

2. Ophthalmo-Operationsmlkroskop (1) nach Anspruch 1, **dadurch gekennzeichnet, dass** für eine stereoskopische Wiedergabe des künstlich erzeugten Bildes zwei separate Umlenkelemente (10) für die Ausspiegelung und zwei separate Umlenkelemente (9) für die Einspiegelung vorgesehen sind, die sich in dem jeweiligen rechten und linken Beobachtungs-Strahlengang (2) des Ophthalmo-Operationsmikroskops (1) befinden.

3. Ophthalmo-Operationsmikroskop (1) nach Anspruch 1 oder 2, **dadurch gekennzeichnet, dass** die Auswertungseinheit (14) dem lichtempfindlichen Sensor (12) zugeordnet und beispielsweise ein Computer ist, der ein Erfassen und Darstellen von Art, Form und richtiger Lage der einzelnen Medien und/oder deren Grenzschichten erlaubt.

4. Ophmalmo-Operationsmikroskop (1) nach einem der vorhergehenden Ansprüche, **dadurch gekennzeichnet, dass** der Projektor (16), der Monitor oder das Display beispielsweise ein Echtfarben-, Falschfarben- oder SchwarzlWeiß-System ist.

5. Ophthalmo-Operationsmikroskop (1) nach einem der vorhergehenden Ansprüche, **dadurch gekennzeichnet, dass** die Lichtquelle (7) aus der folgenden Gruppe ausgesucht Ist: Kohärente oder inkohärente Lichtquelle, Laser, Diode, Lampe.

## Claims

1. Ophthalmic surgical microscope (1) for viewing a patient's eye (5), wherein the ophthalmic surgical microscope (1) has an objective (3), a magnification system, a tube with eyepieces (4), and at least one viewing beam path (2) leading from the patient's eye (5) through the objective (3) and through the tube with eyepieces (4) to a viewer (6)
and wherein a light source (7) whose illumination beam path (8) can be deflected by means of a deflecting element (21) and the objective (3) such that the illumination beam path (8) strikes the patient's eye (5) with the viewing beam path (2) is arranged on the ophthalmic surgical microscope (1), **characterized in that** at least one first spectral, colour or polarization filter (18), or combinations thereof, are arranged in the illumination beam path (8) and at least one second spectral, colour or polarization filter (20) is arranged between the magnification system and the tube with eyepieces (4) or in the tube with eyepieces (4) itself such that they can be switched in, with the result that the different media of the patient's eye can be displayed differently, **in that** a light-sensitive sensor (12) is provided for electronically detecting the illumination light reflected by the patient's eye (5) and output via a deflecting element (10) from the viewing beam path (2), by means of which sensor the reflected illumination light is converted into corresponding electronic signals (13) and passed on to an evaluation unit (14) by means of which the electronic signals of the sensor (12) can be evaluated; and can be passed on to a projector (16), monitor or display, which can be driven, in the operating state, by a drive signal (15) of the evaluation unit (14) such that an image is produced which can, in turn, be reflected, via a deflecting element (9), into the viewing beam path (2) of the ophthalmic surgical microscope (1), and **in that** shutters (17, 22) are arranged in the viewing beam path (2) of the ophthalmic surgical microscope (1) and in the beam path of the input image, which shutters, in the operating state, optionally switch off the direct viewing light or the light reflected in.

2. Ophthalmic surgical microscope (1) according to Claim 1, **characterized in that** two separate deflecting elements (10) for reflecting out and two separate deflecting elements (9) for reflecting in are provided for a stereoscopic reproduction of the artificially produced image and are situated in the respective right-hand and left-hand viewing beam path (2) of the ophthalmic surgical microscope.

3. Ophthalmic surgical microscope (1) according to Claim 1 or 2, **characterized in that** the evaluation unit (14) is assigned to the light-sensitive sensor (12) and is, for example, a computer, which permits acquisition and display of type, shape and correct position of the individual media and/or their interfaces.

4. Ophthalmic surgical microscope (1) according to one of the preceding claims, **characterized in that** the projector (16), the monitor or the display is, for example, a true-colour, false-colour or black/white system.

5. Ophthalmic surgical microscope (1) according to one of the preceding claims, **characterized in that** the light source (7) is selected from the following group: coherent or incoherent light source, laser, diode, lamp.

## Revendications

1. Microscope (1) de chirurgie ophtalmologique pour examiner l'oeil (5) d'un patient, le microscope (1) de chirurgie ophtalmologique présentant un objectif (3), un système d'agrandissement, un tube doté d'oculaires (4), au moins un parcours (2) de rayons d'observation qui sont amenés de l'oeil (5) du patient à un observateur (6) par l'objectif (3) et le tube doté d'oculaires (4), une source de lumière (7) étant disposée sur le microscope (1) de chirurgie ophtalmologique, le parcours (8) de ses rayons d'éclairage pouvant être dévié au moyen d'un élément de déviation (21) et de l'objectif (3) de telle sorte que le parcours (8) des rayons d'éclairage aboutisse sur l'oeil du patient (5) avec le parcours (2) des rayons d'observation,
**caractérisé en ce que**
au moins un premier filtre spectral, coloré ou polarisant (18) ou une combinaison de ces filtres est disposé dans le parcours (8) des rayons d'éclairage et au moins un deuxième filtre spectral, coloré ou polarisant (20) est disposé entre le système d'agrandissement et le tube doté d'oculaires (4) ou peut se raccorder de lui-même dans le tube doté d'oculaires (4), de manière à pouvoir représenter de manières différentes les différents fluides présents dans l'oeil (5) du patient,
**en ce qu'**un détecteur (12) sensible à la lumière et qui détecte électroniquement la lumière d'éclairage réfléchie par l'oeil (5) du patient et extraite du parcours (2) de rayons d'observation par un élément de déviation (10) est prévu et convertit la lumière d'éclairage en signaux électroniques (13) appropriés qui sont transmis à une unité d'évaluation (14) qui permet d'évaluer les signaux électroniques du détecteur (12) et les transmet à un projecteur (16), un écran ou un affichage qui, en utilisation, peuvent être commandés par un signal de commande (15) de l'unité d'évaluation (14) de manière à créer une image qui peut être réfléchie par un élément de déviation (9) dans le parcours (2) des rayons d'observation du microscope (1) de chirurgie ophtalmologique et
**en ce que** des obturateurs (17, 22) qui, en fonctionnement, débranchent sélectivement la lumière directe d'observation ou la lumière réfléchie, sont disposés dans le parcours (2) des rayons d'observation du microscope (1) de chirurgie ophtalmologique et dans le parcours des rayons de l'image séparée.

2. Microscope (1) de chirurgie ophtalmologique selon la revendication 1, **caractérisé en ce que** pour représenter en stéréoscopie l'image créée artificiellement, deux éléments séparés de déviation (10) sont prévus pour la séparation par réflexion et deux éléments séparés de déviation (9) sont prévus pour l'injection dans la réflexion et sont situés respectivement dans le parcours droit et dans le parcours gauche (2) des rayons d'observation du microscope (1) de chirurgie ophtalmologique.

3. Microscope (1) de chirurgie ophtalmologique selon les revendications 1 ou 2, **caractérisé en ce que** l'unité d'évaluation (14) est associée au détecteur (12) sensible à la lumière et est par exemple un ordinateur qui permet de détecter et de présenter la nature, la forme et la position correcte des différents fluides et/ou de leurs couches frontières.

4. Microscope (1) de chirurgie ophtalmologique selon l'une des revendications précédentes, **caractérisé en ce que** le projecteur (16), l'écran ou l'affichage sont par exemple un système en couleurs vraies, en couleurs artificielles ou en noir et blanc.

5. Microscope (1) de chirurgie ophtalmologique selon l'une des revendications précédentes, **caractérisé en ce que** la source de lumière (7) est sélectionnée dans le groupe formé par les sources de lumière cohérente ou incohérente, les lasers, les diodes et les lampes.
